(19) Europäisches Patentamt
European Patent Office
Office européen des brevets

(11) Veröffentlichungsnummer: **0 223 001**
**B1**

(12) # EUROPÄISCHE PATENTSCHRIFT

(45) Veröffentlichungstag der Patentschrift: **29.08.90**

(51) Int. Cl.[5]: **C07C 333/16**

(21) Anmeldenummer: **86112463.4**

(22) Anmeldetag: **09.09.86**

(54) **Verfahren zur Herstellung eines Ammoniak-Komplexes von Zink-bis-dithiocarbamat.**

(30) Priorität: **26.09.85 DE 3534245**

(43) Veröffentlichungstag der Anmeldung: **27.05.87 Patentblatt 87/22**

(45) Bekanntmachung des Hinweises auf die Patenterteilung: **29.08.90 Patentblatt 90/35**

(84) Benannte Vertragsstaaten: **BE DE FR GB IT NL**

(56) Entgegenhaltungen: **DE-B-1 041 031**

(73) Patentinhaber: **AKZO N.V., Velperweg 76, NL-6824 BM Arnhem(NL)**

(72) Erfinder: **Bergfeld, Manfred, Dr. Dipl.-Chem., August-Pfeffer-Strasse 6, D-8765 Erlenbach/Mechenhard(DE)**
Erfinder: **Eisenhuth, Ludwig, Dr. Dipl.-Chem., Lauterhofstrasse 44, D-8753 Obernburg(DE)**

(74) Vertreter: **Fett, Günter, Akzo Patente GmbH Kasinostrasse 19 - 23, D-5600 Wuppertal 1(DE)**

EP 0 223 001 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Ammoniak-Komplexen von Zink-alkylen-bis-dithiocarbamaten ausgehend von Schwefelkohlenstoff, Ammoniak, einem Alkylen-diamin und Zinkoxid.

Derartige Produkte sind als Fungizide bekannt, insbesondere das Zink-ethylen-bis-dithiocarbamat, auch "Zineb" genannt. Nach der DE-PS l2 26 36l kann es auf folgende Weisen hergestellt werden:

a) aus dem Zineb • 2 $NH_3$-Komplex durch Ansäuern,
b) aus einer wäßrigen Zineb- Suspension durch längeres Rühren mit einer wäßrigen Ammoniaklösung,
c) aus einem wasserlöslichen Salz der Ethylen-bis-dithiocarbamidsäure durch Umsatz mit einem wasserlöslichen Zinksalz in Gegenwart von l Mol Ammoniak je Mol Zinksalz.

Aus der österreichischen Patentschrift l9 54 4O ist als Herstellungsmethode außerdem bekannt,

d) eine wäßrige Lösung von Ammonium-ethylen-bis-dithiocarbamat mit Zinkoxid umzusetzen und
e) Zinkoxid mit den nötigen Mengen der zur Bildung des Ammonium-dithio-carbamats erforderlichen Ausgangssubstanzen reagieren zu lassen.

In dieser Patentschrift ist nur von Zink-ethylen-bis-dithiocarbamat, nicht jedoch von dessen Ammoniak-Komplexen die Rede. Es geht auch nicht aus den Beispielen hervor, ob und welche Ammoniak-Komplexe entstehen. Dementsprechend bleibt unklar, was man zu tun hat, um gezielt den Ammoniak-Komplex mit einem Molekül $NH_3$ pro Atom Zink zu erhalten.

Aufgabe der Erfindung ist es, ein Verfahren zur Herstellung von Ammoniak-Komplexen mit Zink-alkylen-bis-dithiocarbamaten mit etwa einem Molekül $NH_3$ pro einem Molekül Zink-dithiocarbamat zu entwickeln, wobei das Produkt in möglichst hohem Reinheitsgrad und auf einfache und wirtschaftlich günstige Weise hergestellt werden soll.

Die erfindungsgemäße Lösung dieser Aufgabe ist den Patentansprüchen zu entnehmen. Die Erfindung ist im wesentlichen dadurch gekennzeichnet, daß man Alkylendiamin, Schwefelkohlenstoff und Ammoniak im Molverhältnis von l/2/weniger als 2 in Wasser mischt und zu dieser Lösung Zinkoxid hinzufügt. Es ist also nicht notwendig, daß Ammoniak in der zur Bildung von Ammonium-alkylen-bis-dithiocarbamat notwendigen Menge von 2 Molen $NH_3$ pro 2 Mol $CS_2$ bzw. l Mol Alkylendiamin eingesetzt wird, wie die AT-PS l 954 44O lehrt.

Die Reaktion läßt sich z.B. durch folgende Gleichung summarisch darstellen:

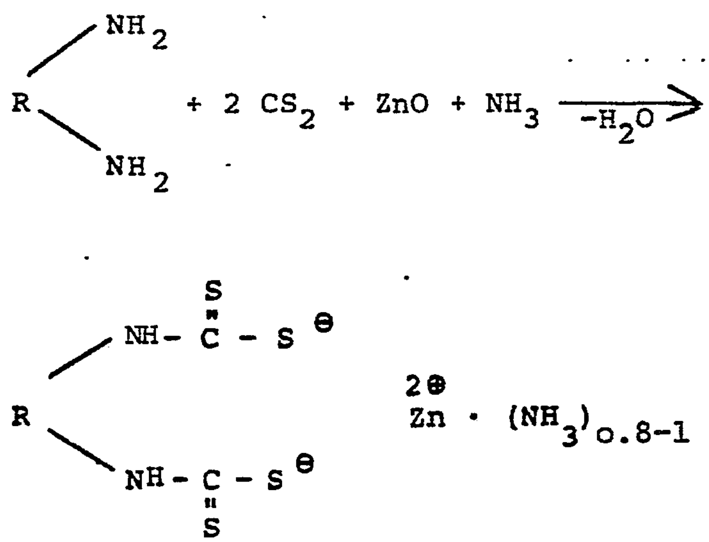


R = $C_nH_{2n}$ (n = 2 - 6)

Die allgemeine Arbeitsweise zeichnet sich durch ihre Einfachheit aus und stellt sich wie folgt dar:

In einem Reaktionsgefäß wird zu einer wäßrigen Lösung von Alkylendiamin und Ammoniak unter intensivem Rühren der Schwefelkohlenstoff addiert, wobei die Temperatur unter 3O°C gehalten wird. Dazu gibt man das Zinkoxid und rührt weiter, wobei die Reaktionstemperatur im Bereich von 3O bis 6O°C liegen

kann. Je nach Reaktionstemperatur und Rührintensität liegt die Reaktionszeit bei l - 6 h. Das Reaktionsprodukt wird abfiltriert, ggf. mit Wasser gewaschen und getrocknet.

Bevorzugt werden 2 Mol Schwefelkohlenstoff, sowie O.6 - l.5 Mol Ammoniak pro Mol Alkylendiamin eingesetzt. Das Zinkoxid wird bevorzugt stöchiometrisch eingesetzt, kann aber auch in geringen Mengen eingesetzt werden. Im Überschuß eingesetztes Zinkoxid wird dagegen (als Verunreinigung) im Produkt erscheinen.

Der Ammoniak-Gehalt der so erhaltenen Zink-dithiocarbamat-Produkte hängt von der eingesetzten Ammoniak-Menge ab; im allgemeinen liegt der Ammoniak-Gehalt bei etwa O.8 bis l Mol pro Mol Zink-dithiocarbamat.

Die erhaltenen Produkte werden durch IR-Messung, Elementar-Analyse, $CS_2$-Bestimmung nach der Methode aus CIPAC Handbock, l97O, l, S. 7O6 und $NH_3$-Bestimmung (titrimetrische Bestimmung des mit Natronlauge freizusetzenden Ammoniaks) charakterisiert.

Nach dem Verfahren wurden Ausbeuten bis zu 97% erzielt, sowie Produktreinheiten bis zu 97%.

Folgende Ausführungsformen werden bevorzugt:

Als Diamin können aliphatische Diamine mit 2 bis 6 C-Atomen eingesetzt werden.

Vorzugsweise wird ein aliphatisches l,2-Diamin, insbesondere Ethylendiamin oder Propylendiamin verwendet.

Es ist vorteilhaft, wenn die Umsetzung unter intensivem Rühren in einem wäßrigen Medium durchgeführt wird. Vorteilhaft ist auch die Gegenwart grenzflächenaktiver Mittel.

Für das Verfahren wurde handelsübliches Zinkoxid ohne jede Vorbehandlung eingesetzt. Erwartungsgemäß ist es vorteilhaft, möglichst feinkörniges Zinkoxid (Körnung < O.l mm) zu verwenden.

Es war überraschend, daß man gemäß der Erfindung auf einfache und wirtschaftliche Weise den Ammoniak-Komplex der Zink-alkylen-bis-dithiocarbamate durch Einsatz von Schwefelkohlenstoff und Ammoniak im Molverhältnis von 2 :< 2 erhalten konnte, daß also nicht die zur Bildung des Ammonium-dithiocarbamats erforderlichen Mengen an Ausgangssubstanzen notwendig sind. Trotz des geringeren Ammoniak-Gehaltes werden höhere Ausbeuten und bessere Produktqualitäten erzielt. Außerdem entfällt die vorausgehende, aufwendige Synthese der Ammonium-ethylen-bis-dithiocarbamat-Zwischenstufe.

Darüberhinaus hat das erfindungsgemäße Verfahren den Vorteil, daß die Mutterlauge im Kreislauf geführt werden kann, was eine beachtliche Materialeinsparung sowie eine starke Verringerung der Abwasserbelastung mit sich bringt.

Das erhaltene Produkt kann direkt als Fungizid verwendet werden. Es ist auch möglich, die Produkte mit anderen Verbindungen zu verschneiden, wie zum Beispiel mit Mangan-ethylen-bis-dithiocarbamat. Das erhaltene Produkt kann auch mit Formaldehyd vernetzt werden.

Die Erfindung wird durch folgende Beispiele näher erläutert:

Beispiel l:

In einem Glasreaktionsgefäß wird zu einer Lösung aus O,5 Mol Ethylendiamin und O,5 Mol Ammoniak in 2OO g Wasser, sowie wenigen Tropfen Serdox NOP 9 [Nonylphenylpolyethylenglykol (Chemische Fabrik Servo b.v. Delden/NL)] l Mol Schwefelkohlenstoff unter intensivem Rühren addiert, wobei die Temperatur unter 3O°C gehalten wurde. Nach l5 Min fortgesetztem Rühren wurden O,5 Mol Zinkoxid hinzugefügt und 2,5 h bei 4O°C nachgerührt. Der entstandene weiße Niederschlag wurde abfiltriert, mit Wasser gewaschen und bei 5O°C getrocknet. Man erhält auf diese Weise l35,4 g eines Produktes, das in seiner Infrarotanalyse (Abb. l) identisch ist mit dem Ammoniak-Komplex des Zinkethylen-bis-(dithiocarbamat) ("Zineb"), das in der DE-PS l 226 36l beschrieben ist. Die Ausbeute an Zineb•$NH_3$ beträgt 92,6% d. Th., die Produktreinheit liegt bei 96,l% (bestimmt über $CS_2$-Analyse).

| Analyse: | gefunden % | berechnet % |
|---|---|---|
| C | 16,6 | 16,4 |
| H | 3,1 | 3,1 |
| N | 14,0 | 14,4 |
| $CS_2$ | 50,0 | 52,0 |
| $NH_3$ | 5,0 | 5,8 |

Die Mutterlauge enthält noch kleine Mengen der nicht umgesetzten Ausgangsstoffe Schwefelkohlenstoff, Ethylendiamin und Ammoniak und kann daher erneut eingesetzt werden.

Beispiel 2 (Vergleichsbeispiel):

In diesem Beispiel wird wie in Beispiel 4 der österreichischen Patentschrift Nr. l95 44O gearbeitet.

In einem Glaskolben werden 4O,7 g ZnO (Reinheit > 99%) eingetragen und 25O ml O,3 g ligninsulfosaures Natron enthaltendes Wasser zugesetzt. Diese Aufschlämmung wird l5 Minuten lang durchgeführt.

Sodann werden rasch 82 g Schwefelkohlenstoff eingetragen. Es wird einige Minuten durchgerührt und dann ein aus 39 g Ethylendiamin und 68 g wäßrigem Ammoniak (25 Gew.-%ig) bestehendes Gemisch tropfenweise und unter ununterbrochenem Durchrühren im Laufe einer Stunde zugesetzt. Die Temperatur wird durch ein Wasserbad auf 3O bis 35°C gehalten. Nach Beendigung des Zusatzes der beiden Basen läßt man noch während weiterer drei Stunden reagieren. Das Produkt wird filtriert, mit Wasser gewaschen und bei 5O°C getrocknet.

Ausbeute: I44,6 g. Laut IR-Spektrum (Abb. 2) liegt weder Zineb noch Zineb • $NH_3$ vor. Auch die Analysenwerte weichen stark von den für Zineb • $NH_3$ berechneten Werten ab. Die Struktur der Substanz wurde nicht aufgeklärt.

| Analyse: | gefunden % |
|---|---|
| C | 18,0 |
| H | 3,8 |
| N | 15,5 |
| $CS_2$ | 43,2 |
| $NH_3$ | 5,1 |

Dieses Verfahren führt demnach nicht zu dem gewünschten Zineb • $NH_3$-Produkt.

Beispiel 3:

Wie in Beispiel I beschrieben, werden 2 Mol Ethylendiamin, 4 Mol Schwefelkohlenstoff, 2 Mol Ammoniak in 68O g Wasser mit 2 Mol Zinkoxid zur Reaktion gebracht. Nach der Zugabe des Zinkoxids wird noch I h bei 3O°C und I h bei 4O°C weitergerührt. Das Zineb.$NH_3$-Produkt wird dabei in 554,7 g Ausbeute (entsprechend 94,8% d.Th.) erhalten; Gehalt an Zineb $NH_3$ liegt bei 93,4%.

Beispiel 4 (Vergleichsbeispiel)

In diesem Beispiel wird zunächst Ammoniumethylen-bis-(dithiocarbamat) hergestellt und dieses entsprechend dem österreichischen Patent I95 44O (Beispiel I) mit ZnO umgesetzt.

Die Herstellung des Ammoniumethylen-bis-(dithiocarbamats) erfolgte nach dem im US-Patent 2 844 623 beschriebenen Verfahren. 4 Mol Ammoniak und 2 Mol Ethylendiamin werden in 4IO g Wasser mit wenigen Tropfen Serdox NOP 9 vorgelegt und während I h unter Rühren 4 Mol Schwefelkohlenstoff zugetropft (Temperatur: 3O - 35°C). Anschließend wird eine weitere Stunde bei 35 - 38°C gerührt.

Analog Beispiel I der AT-PS I95 44O werden in einem zweiten Reaktionsgefäß 2 Mol Zinkoxid IO Min lang in 54O g Wasser aufgeschlämmt. Zu dieser Aufschlämmung wird bei Raumtemperatur die oben hergestellte Ammoniumethylen-bis-(dithiocarbamat)-Lösung zugesetzt und I h nachgerührt. Die Temperatur wird bei 28 - 3O°C gehalten. Man gewinnt auf diese Weise 479,5 g eines Produkts, das in seiner IR-Analyse dem Zineb•$NH_3$-Komplex entspricht (Ausbeute: 8O,I% d. Th., Gehalt an Zineb•$NH_3$ 9O,2%).

Beispiele 5 bis 7:

Es wird wie in Beispiel I gearbeitet, aber die eingesetzte Ammoniak-Menge wird variiert. Die Ammoniak-Menge sowie die erhaltenen Ergebnisse sind der folgenden Tabelle zu entnehmen.

| Beispiel | $NH_3$ Mol | Produkt g | Zineb/$NH_3$-Gehalt % | $NH_3$ Gew.-% |
|---|---|---|---|---|
| 5 | 0,75 | 141 | 95,8 | 5,4 |
| 6 | 0,4 | 134 | 95,5 | 4,6 |
| 7 | 0,3 | 126 | 94,4 | 3,7 |

Beispiel 8:

I Mol Propylendiamin und I,5 Mol Ammoniak werden in 25O g Wasser vorgelegt. Dazu werden bei 2O - 3O°C 2 Mol Schwefelkohlenstoff gegeben und nach 3O-minütigem, intensivem Durchmischen I Mol Zinkoxid zugesetzt. Abschließend wird 3 h bei 35°C nachgerührt, der leicht gelbliche Niederschlag abgesaugt, gewaschen und getrocknet. So werden 288,3 g eines Produktes erhalten, das in seiner Analyse

dem Ammoniak-Komplex des Zink-propylendiamin-bis-(dithiocarbamat) (Gehalt aufgrund der $CS_2$-Analyse: 93,l%) entspricht.

| Analyse: | gefunden % | berechnet % |
|---|---|---|
| C | 19,1 | 19,6 |
| H | 3,7 | 3,6 |
| N | 13,9 | 13,7 |
| $CS_2$ | 46,1 | 49,6 |
| $NH_3$ | 4,8 | 5,5 |

## Patentansprüche

l. Verfahren zur Herstellung von Ammoniak-Komplexen von Zink-alkylene-bis-dithiocarbamaten ausgehend von Schwefelkohlenstoff, Ammoniak, einem Alkylen-diamin und Zinkoxid, dadurch gekennzeichnet, daß ein wäßriges Gemisch aus Alkylendiamin, Schwefelkohlenstoff und Ammoniak im Molverhältnis l : 2 : < 2 mit Zinkoxid umgesetzt wird.

2. Verfahren nach Anspruch l, dadurch gekennzeichnet, daß O.6 - l.5 Mol Ammoniak pro l Mol Alkylendiamin bzw. 2 Mol Schwefelkohlenstoff eingesetzt werden.

3. Verfahren nach Anspruch l oder 2, dadurch gekennzeichnet, daß das Alkylendiamin ein aliphatisches l,2-Diamin mit 2 bis 6 C-Atomen mit primären Aminogruppen ist.

4. Verfahren nach Anspruch l oder 2, dadurch gekennzeichnet, daß das Alkylendiamin Ethylendiamin ist.

5. Verfahren nach Anspruch l oder 2, dadurch gekennzeichnet, daß das Alkylendiamin Propylendiamin ist.

## Claims

1. Process for the preparation of ammonia complexes of zinc alkylene-bis-dithiocarbamates starting from carbon disulphide, ammonia, an alkylene diamine and zinc oxide, characterised in that an aqueous mixture of alkylene diamine, carbon disulphide and ammonia in a molar ratio of 1 : 2 : < 2 is reacted with zinc oxide.

2. Process according to Claim 1, characterised in that from 0.6 to 1.5 mol of ammonia is used per 1 mol of alkylene diamine and 2 mol of carbon disulphide.

3. Process according to Claim 1 or 2, characterised in that the alkylene diamine is an aliphatic 1,2-diamine having 2 to 6 carbon atoms and containing primary amino groups.

4. Process according to Claim 1 or 2, characterised in that the alkylene diamine is ethylene diamine.

5. Process according to Claim 1 or 2, characterised in that the alkylene diamine is propylene diamine.

## Revendications

1. Procédé de préparation de complexes d'ammoniac d'alkylène-bis-dithiocarbamates de zinc, à partir de disulfure de carbone, d'ammoniac, d'une alkylène-diamine et d'oxyde de zinc, caractérisé en ce que l'on fait réagir un mélange aqueux d'alkylène-diamine, de disulfure de carbone et d'ammoniac, en des proportions molaires de 1 : 2 : (moins de 2), avec de l'oxyde de zinc.

2. Procédé selon la revendication 1, caractérisé en ce que l'on utilise de 0,6 à 1,5 mole d'ammoniac pour 1 mole d'alkylène-diamine ou 2 moles de disulfure de carbone.

3. Procédé selon la revendication 1 ou 2, caractérisé en ce que l'alkylène-diamine est une 1,2-diamine aliphatique, comportant de 2 à 6 atomes de carbone et des groupes amines primaires.

4. Procédé selon la revendication 1 ou 2, caractérisé en ce que l'alkylène-diamine est l'éthylène-diamine.

5. Procédé selon la revendication 1 ou 2, caractérisé en ce que l'alkylène-diamine est la propylène-diamine.

Abb. 1: IR-Spektrum: Produkt aus Beispiel 1 (Zineb•$NH_3$)

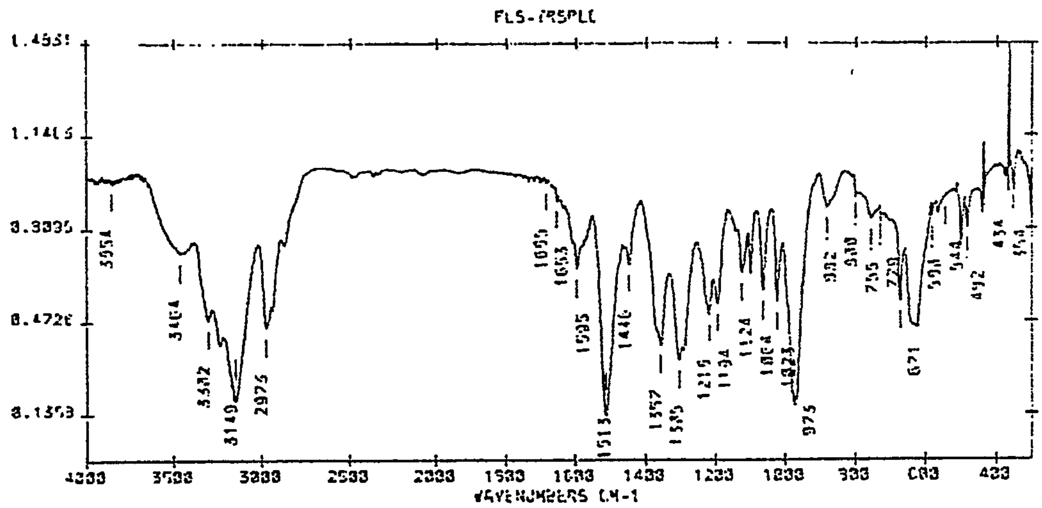


Abb. 2: IR-Spektrum: Produkt aus Vergleichsbeispiel 2 (AT-PS 195 440)